(19) Europäisches Patentamt
European Patent Office
Office européen des brevets

(11) Veröffentlichungsnummer: **0 113 009**
A2

(12) # EUROPÄISCHE PATENTANMELDUNG

(21) Anmeldenummer: **83111460.8**

(22) Anmeldetag: **17.11.83**

(51) Int. Cl.³: **A 61 K 9/06**
A 61 K 31/415, A 61 K 47/00

(30) Priorität: **27.11.82 DE 3244027**

(43) Veröffentlichungstag der Anmeldung:
**11.07.84 Patentblatt 84/28**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **von Bittera, Miklos**
**Max-Scheler-Strasse 7**
**D-5090 Leverkusen 3(DE)**

(72) Erfinder: **Hoff, Dieter, Dr.**
**Edmund-Husserl-Strasse 19**
**D-5090 Leverkusen 3(DE)**

(72) Erfinder: **Büchel, Karl Heinz, Prof. Dr.**
**Dabringhausener Strasse 42**
**D-5093 Burscheid(DE)**

(72) Erfinder: **Plempel, Manfred, Dr.**
**Zwengenberger Strasse 3c**
**D-5657 Haan(DE)**

(72) Erfinder: **Regel, Erik**
**Untere Bergerheide 26**
**D-5600 Wuppertal 1(DE)**

(54) **Antimykotische Mittel mit hoher Wirkstoff-Freisetzung in Form von Gelsystemen.**

(57) Antimykotische Mittel enthalten außer Azolderivaten und üblichen Formulierungshilfsstoffen Benzylalkohol, Spreitmittel und Gelbildner.

EP 0 113 009 A2

Croydon Printing Company Ltd.

BAYER AKTIENGESELLSCHAFT 5090 Leverkusen, Bayerwerk

Zentralbereich
Patente, Marken und Lizenzen Si/ABc

Antimykotische Mittel mit hoher Wirkstoff-Freisetzung in Form von Gelsystemen

Die vorliegende Erfindung betrifft neuartige Gelformulierungen der bekannten antimykotischen Azolderivate, die eine höhere Freisetzung der Wirkstoffe aufweisen und dadurch eine Kurzzeittherapie ermöglichen.

Für die Behandlung von Mykosen beim Menschen, vor allem die Mykosen der Haut sind bereits Zubereitungen von antimykotischen Derivaten bekannt geworden. Mit diesen Zubereitungen wurden für eine vollständige Sanierung 14 bis 21 Tage Therapiezeit benötigt.

Um zu einer Verkürzung der Therapiedauer zu kommen, benötigt man, besonders zu Eliminierung der Keime, bzw. um eine mykologische Sanierung zu erzielen, eine gewisse Depot-Wirkung und eine höhere Bioverfügbarkeit der Wirkstoffe. Dafür sind die bekannten Formulierungen nur begrenzt geeignet, weil sich von dem vorhandenen Wirkstoffangebot nur ein kleiner Anteil im Flüssigkeitsvolumen am Ort der Infektion löst. Wenn man nun durch oder ohne weitere Erhöhung der Wirkstoffkonzentration eine Verkürzung der Therapiedauer erreichen will, muß man für eine optimale Bioverfügbarkeit des Wirkstoffes Sorge tragen.

Es wurde nun gefunden, daß solche Gelformulierungen antimykotischer Azolderivate, die neben üblichen Formulierungsstoffen 1-5 % Benzylalkohol, 2,5-35 % Spreitmittel und Gelbildner enthalten, den Wirkstoff in höherem Maße freisetzen und dadurch eine Verkürzung der Therapiedauer auf 1 Tag ermöglichen. Dieser Effekt der höheren Wirkstoff-Freisetzung kann bis zu einer Zehnerpotenz erreichen.

Wirkstoffe, die in dieser Weise formuliert werden können, sind alle antimykotisch wirksamen Derivate, insbesondere Imidazol- und Triazolderivate. Sie sind in den erfindungsgemäßen Mitteln in Mengen von 0,05 % bis 1 %, vorzugsweise 0,1 bis 1 % vorhanden.

Beispielsweise seien die Verbindungen der nachstehenden Formeln genannt:

I

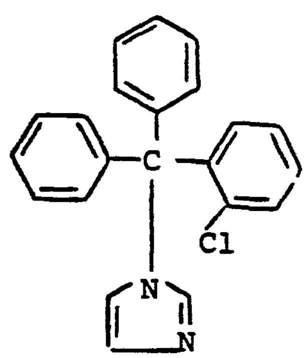

Clotrimazol

II 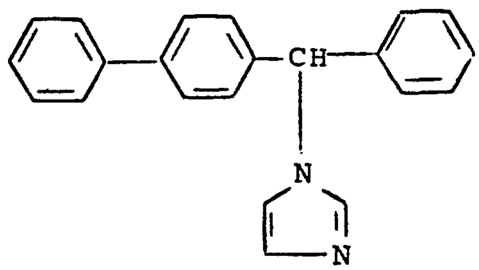 Bifonazol

III 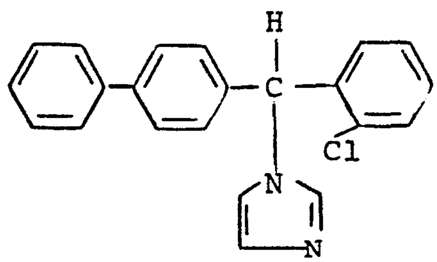 Lombazol

Zahlreiche weitere antimykotisch wirksame Azolderivate sind bekannt aus der DE-OS 24 30 039. Sie können ebenfalls in den erfindungsgemäßen Mitteln als Wirkstoffe dienen.

Unter Spreitmitteln werden ölige Flüssigkeiten verstanden, die sich auf der Haut besonders gut verteilen. (R. Keymer, Pharm. Ind. 32 (1970), 577-581). Für die erfindungsgemäßen Mittel eignen sich als Spreitmittel insbesondere folgende Verbindungen:

Siliconöle verschiedener Viskosität.

Fettsäureester, wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Isopropylisostearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische, Polyol-Fettsäureester u.a.

Triglyceride, wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monoglyceride der $C_8$/$C_{10}$-Fettsäuren u.a.

Fettalkohole, wie Isotridecylalkohol, 2-Octyldodecanol, Cetylstearyl-Alkohol, Oleylalkohol.

Fettsäuren, wie z.B. Ölsäure.
Besonders gut geeignete spreitende Öle sind die folgenden: Isopropylmyristat, Isopropylpalmitat, Capryl/Caprinsäureester von gesättigten Fettalkoholen der Kettenlänge $C_{12}$-$C_{18}$, wachsartige Fettsäureester wie künstliches Entenbürzeldrüsenfett, Silikonöle, Isopropylmyristat/Isopropylstearat/Isopropylpalmitat-Gemisch, Isopropylstearat und Isopropylisostearat.

Folgende weitere Hilfs- und/oder Formulierungs-Grundhilfsstoffe können bei der Herstellung der erfindungsgemäßen Mittel verwendet werden:

Glycerin, Paraffin dickflüssig, Paraffin dünnflüssig, Triethanolamin, Collagen, Allantoin, Novantisolsäure, Parfümöle.

Als Gelbildner verwendet man Cetylstearylalkohol mit ca. 30 Mol Ethylenoxid, gegebenenfalls in Gemisch mit Polyol-Fettsäureestern, in Konzentrationen von 10-30 %.

Als weitere Gelbildner kommen noch solche makromolekularen Verbindungen in Frage, die sich sowohl in Wasser als auch in organischen Lösungsmitteln lösen bzw. anquellen können.

Folgt man einer Einteilung der makromolekularen Hilfsstoffe, (Keipert et al., Die Pharmazie 28, 145-183 (1973)), so kommen vor allem ionische Makromoleküle und deren Salzform zur Anwendung. Dies sind unter anderem Polyacrylsäure, Polymethacrylsäure und deren Salze, wie z.B. eine schwach vernetzte Polyacrylsäure von extrem hohem Molgewicht, die durch Zusatz eines Alkalis eine klare Gelstruktur bildet. Im Falle der Polymethacrylsäure und/oder deren Salzen werden 1-5 % benötigt.

Als Lösungsmittel sind Wasser und auch alle mit Wasser mischbaren Lösungsmittel geeignet. In Betracht kommen z.B. Alkanole wie Ethanol und Isopropylalkohol, Benzylalkohol, Propylenglykol, Methylcellosolve, Cellosolve,

Ester, Morpholine, Dioxan, Dimethylsulfoxid, Dimethylformamid, Tetrahydrofuran, Cyclohexanon.

Es können bei der Herstellung der erfindungsgemäßen Formulierungen ein oder mehrere Lösungsmittel eingesetzt werden.

Die erfindungsgemäßen antimykotischen Gele werden dadurch hergestellt, daß man ein Gemisch aus einem oder mehreren antimykotischen Azolderivaten, 1 - 5 % Benzylalkohol, 2,5 - 35 % Spreitmittel und als Gelbildner 10 - 30 % Cetylstearylalkohol mit ca. 30 Mol. Ethylenoxid bei 110 °C schmilzt, bei Stickstoffbegasung unter Druck mit der Wasserphase (100 °C) vereinigt und unter langsamen Rühren auf 25 °C abkühlt.

Das Azolderivat wird dabei in Menge von 0.05 - 1 %, vorzugsweise 0.1 - 1 %, eingesetzt.
Die Wasserphase besteht aus 35 - 55 % entmineralisiertem Wasser.

Beispiel 1

Phase I

| | |
|---|---|
| Clotrimazol | 1,000 g |
| Polyol-Fettsäureester | 23,000 g |
| Cetylstearylalkohol, mit ca. 30 Mol Ethylenoxid | 16,000 g |
| Isopropylmyristat | 10,000 g |
| Benzylalkohol | 3,000 g |

Die Mischung wird unter Rühren auf 100°C erwärmt.

Phase II

| | |
|---|---|
| Wasser entmineralisiert auf 100°C erwärmen | 47,00 g |

Phase II in Phase I bei mindestens 95°C langsam einrühren. Bei 60°C eventuellen Wasserverlust ausgleichen und bis 50°C langsam weiterrühren. Ab 50°C ohne Rühren auf 25°C abkühlen (um Lufteinschlüsse zu vermeiden bzw. die Gelbildung nicht negativ zu beeinflussen).

In analoger Weise wird bei den Beispielen 2-6 gearbeitet.

Beispiel 2

| | |
|---|---|
| Bifonazol | 1,00 g |
| Polyol-Fettsäureester | 20,00 g |

| | |
|---|---|
| Cetylstearylalkohol, | |
| mit ca. 30 Mol Ethylenoxid | 16,00 g |
| Isopropylmyristat | 10,00 g |
| Benzylalkohol | 3,00 g |
| Milchsäure | 1,50 g |
| Wasser entmineralisiert | 48,50 g |

Beispiel 3

| | |
|---|---|
| Clotrimazol | 1,00 g |
| Polyol-Fettsäureester | 20,00 g |
| Cetylstearylalkohol, | |
| mit ca. 30 Mol Ethylenoxid | 20,00 g |
| Isopropylisostearat | 5,00 g |
| Ethanol 96 %ig | 3,00 g |
| Benzylalkohol | 1,00 g |
| Wasser entmineralisiert | 50,00 g |

Beispiel 4

| | |
|---|---|
| Bifonazol | 1,00 g |
| Polyol-Fettsäureester | 23,00 g |
| Cetylstearylalkohol, | |
| mit ca. 30 Mol Ethylenoxid | 12,00 g |
| Isopropylmyristat | 10,00 g |
| Benzylalkohol | 3,00 g |
| Wasser entmineralisiert | 51,00 g |

Beispiel 5

| | |
|---|---|
| Lombazol | 1,00 g |
| Polyol-Fettsäureester | 20,00 g |
| Cetylstearylalkohol, mit ca. 30 Mol Ethylenoxid | 20,00 g |
| Isopropylisostearat | 5,00 g |
| Ethanol | 3,50 g |
| Benzylalkohol | 1,00 g |
| Wasser entmineralisiert | 49,50 g |

Beispiel 6

| | |
|---|---|
| Bifonazol | 1,00 g |
| Polyol-Fettsäureester | 20,00 g |
| Cetylstearylalkohol, mit ca. 30 Mol Ethylenoxid | 20,00 g |
| Isopropylmyristat | 5,00 g |
| Benzylalkohol | 5,00 g |
| Wasser entmineralisiert | 49,00 g |

Beispiel 7

Phase I

| | |
|---|---|
| Clotrimazol | 1,00 g |
| in Isopropanol | 40,00 g |
| lösen; anschließend | |
| Polyol-Fettsäureester, | 4,00 g |
| Benzylalkohol, | 3,00 g |
| Adipinsäurediisopropylester | 4,00 g |
| einrühren und lösen. | |

Phase II

| | |
|---|---|
| in entmineralisiertes Wasser | 46,10 g |
| Carbopol 940 | 1,50 g |
| unter Rühren einbringen, | |
| ca. 2 h quellen lassen und mit | |
| NaOH 45 %ig | 0,40 g neutralisieren, |

Phase I, portionsweise, langsam unter Rühren in Phase II einarbeiten.

In analoger Weise wird bei den Beispielen 8 und 9 gearbeitet.

Beispiel 8

| | |
|---|---|
| Lombazol | 1,00 g |
| Polyol-Fettsäureester | 4,00 g |
| Isopropylmyristat | 4,00 g |
| Benzylalkohol | 1,00 g |
| Isopropanol | 45,00 g |
| Carbopol 940 | 1,50 g |
| NaOH 45 %ig | 0,40 g |
| Wasser entmineralisiert | 43,10 g |

Beispiel 9

| | |
|---|---|
| Bifonazol | 1,00 g |
| Polyol-Fettsäureester | 4,00 g |

| | |
|---|---|
| Kokosfettsäure-Isopropylester | 4,00 g |
| Benzylalkohol | 5,00 g |
| Isopropanol | 45,00 g |
| Carbopol 940 | 1,50 g |
| NaOH 45 %ig | 0,40 g |
| Wasser entmineralisiert | 39,10 g |

Wirksamkeitstestung der erfindungsgemäßen Mittel am Trichophyton-infizierten Meerschweinchen.

Als Testmodell zur vergleichenden Wirksamkeitsprüfung der erfindungsgemäßen Formulierungen verwendeten wir Trichophyton-infizierte Pirbright-white-Meerschweinchen mit einem durchschnittlichen Gewicht von 600 g. Die Tiere wurden auf dem Rücken mit einer elektrischen Haarschneidemaschine so geschoren, daß ca. 1/10 mm lange Haarstümpfe stehen blieben.

Die Infektion mit Trichophyton mentagrophytes erfolgte durch leichtes Verreiben einer 24 Stunden in Sabouraud-Nährlösung angekeimten Sporensuspension des Erregers auf einer ca. 2 x 2 cm großen Fläche des geschorenen Rückens der Tiere. Aufgetragen wurden pro Tier 0,5 ml Keimsuspension, die 1 - 3 x $10^5$ infektiöse Pilzpartikel enthielten.

Bei diesem Infektionsmodus zeigen sich 2 - 3 Tage post infectionem die ersten Symptome der Dermatophytose als Rötung und Schuppung der Haut. Bei unbehandelten Tieren ist ca. 14 Tage p.i. die Dermatophytose maximal ausgeprägt: flächiger Haarausfall und blutige Integument-Defekte innerhalb einer entzündlich veränderten, schuppigen Randzone.

Die zu prüfenden Formulierungen wurden 1-mal, am 2. Tag post infectionem, lokal auf die gerötete Infektionsstelle der Tiere appliziert und mit einem Hornspatel leicht verrieben.

Es wurden jeweils 0,5 g der Formulierungen = 5 mg Wirkstoff aufgetragen. Die Bewertung des Infektionsablaufs erfolgte täglich bis zum 20. Tag p.i.

Die Testergebnisse sind aus der nachstehenden Tabelle ersichtlich.

| Mittel des Beispiels | Wirkung am Trichophyton-infizierten Meerschweinchen |
|---|---|
| 1 | **** |
| 2 | **** |
| 3 | **** |
| 4 | **** |
| 5 | **** |
| 6 | **** |
| 7 | **** |
| 8 | **** |
| 9 | **** |

****: sehr gute Wirkung
*** : gute Wirkung
**: Wirkung
* : schwache Wirkung
0 : keine Wirkung

Verwendet man anstelle der erfindungsgemäßen Formulierungen solche, die keinen Benzylalkohol und kein Spreitmittel enthalten, so wird ein Effekt, der dem der erfindungsgemäßen Formulierungen entspricht, erst nach dreimaliger Applikation erreicht.

Patentansprüche

1. Antimykotische Gele mit höherer Freisetzung der Wirkstoffe, enthaltend Azolderivate und übliche Formulierungshilfsstoffe, dadurch gekennzeichnet, daß sie 1-5 % Benzylalkohol, 2,5-35 % Spreitmittel und als Gelbildner entweder

   a) 10-30 % Cetylstearylalkohol, mit ca. 30 Mol Ethylenoxid oder

   b) 1-5 % Polyacrylsäure, Polymethacrylsäure und/oder Salze

   enthalten.

2. Antimykotische Gele nach Anspruch 1, dadurch gekennzeichnet, daß sie als Wirkstoff Clotrimazol der Formel

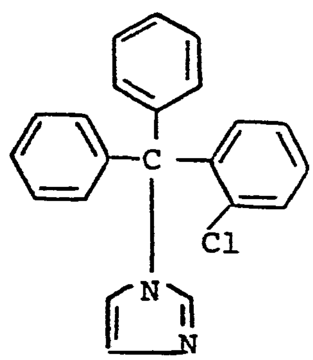

   enthalten.

3. Antimykotische Gele nach Anspruch 1, dadurch gekennzeichnet, daß sie als Wirkstoff die Bifonazol der Formel

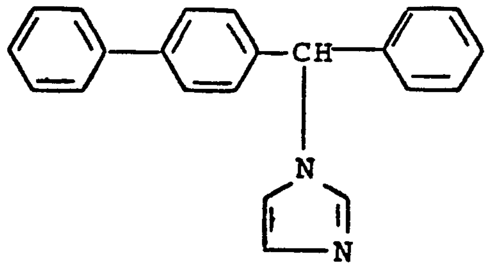

enthalten.

4. Antimykotische Gele nach Anspruch 1, dadurch gekennzeichnet, daß sie als Wirkstoff Lombazol der Formel

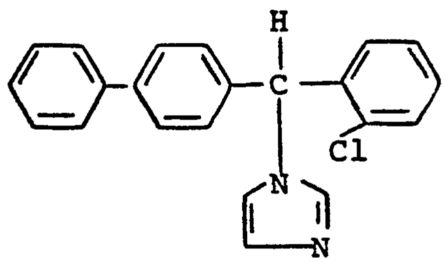

enthalten.

5. Antimykotische Gele nach Anspruch 1, dadurch gekennzeichnet, daß sie die antimykotischen Azolderivate in Mengen von 0,05-1 %, vorzugsweise von 0,1-1 % enthalten.

6. Verfahren zur Herstellung von antimykotischen Gelen, dadurch gekennzeichnet, daß man ein Gemisch aus einem oder mehreren antimykotischen Azolderivaten, 1 - 5 % Benzylalkohol, 2,5 - 35 % Spreitmittel und als Gelbildner 10 - 30 % Cetylstearylalkohol mit ca. 30 Mol. Ethylenoxid bei 110 °C schmilzt, bei Stickstoffbegasung unter Druck mit der Wasserphase (100 °C) vereinigt und unter langsamen Rühren auf 25 °C abkühlt.

7. Verfahren zur Herstellung von antimykotischen Gelen, dadurch gekennzeichnet, daß man ein Gemisch aus Clotrimazol, 1 - 5 % Benzylalkohol, 2,5 - 35 % Spreitmittel und als Gelbildner 10 - 30 % Cetylstearylalkohol mit ca. 30 Mol. Ethylenoxid bei 110 °C schmilzt, bei Stickstoffbegasung unter Druck mit der Wasserphase (100 °C) vereinigt und unter langsamen Rühren auf 25 °C abkühlt.

8. Verfahren zur Herstellung von antimykotischen Gelen, dadurch gekennzeichnet, daß man ein Gemisch aus Bifonazol, 1 - 5 % Benzylalkohol, 2,5 - 35 % Spreitmittel und als Gelbildner 10 - 30 % Cetylstearylalkohol mit ca. 30 Mol. Ethylenoxid bei 110 °C schmilzt, bei Stickstoffbegasung unter Druck mit der Wasserphase (100 °C) vereinigt und unter langsamen Rühren auf 25 °C abkühlt.

9. Verfahren zur Herstellung von antimykotischen Gelen, dadurch gekennzeichnet, daß man ein Gemisch aus Lombazol, 1 - 5 % Benzylalkohol, 2,5 - 35 % Spreitmittel und als Gelbildner 10 - 30 % Cetylstearylalkohol mit ca. 30 Mol. Ethylenoxid bei 110 °C schmilzt, bei Stickstoffbegasung unter Druck mit der Wasserphase (100 °C) vereinigt und unter langsamen Rühren auf 25 °C abkühlt.

10. Verfahren zur Herstellung von antimykotischen Gelen, dadurch gekennzeichnet, daß man ein Gemisch aus Climbazol, 1 - 5 % Benzylalkohol, 2,5 - 35 % Spreitmittel und als Gelbildner 10 - 30 % Cetylstearylalkohol mit ca. 30 Mol. Ethylenoxid bei 110 °C schmilzt, bei Stickstoffbegasung unter Druck mit der Wasserphase (100 °C) vereinigt und unter langsamen Rühren auf 25 °C abkühlt.